# EUROPEAN PATENT APPLICATION

(11) **EP 1 637 154 A2**
(43) Date of publication of application: **22.03.2006**
(21) Application number: 05025878.9
(22) Date of filing: 01.07.1999
(51) Int. Cl.: A61K 38/00, A61K 9/14

(54) **High molecular weight medicine-containing preparation in powder form for administration through mucosa**

(30) Priority: 08.07.1998 JP 19272298; 25.03.1999 JP 8154999
(62) Divisional of application: 99926887.3
(71) Applicant: Kirin-Amgen, Inc., Wilmington, DE 19801 (US)
(72) Inventor: Nomura, Hideaki / Iyaku Kaihatsu Kenkyusho, Takasaki-shi Gunma 370-1295 (JP); Ueki, Yosuke / Iyaku Kaihatsu Kenkyusho, Takasaki-shi Gunma 370-1295 (JP)
(74) Representative: HOFFMANN EITLE

(57) **Abstract**

A preparation in powder form for administration through mucosa, comprising a medicine of high molecular weight and a cationic polymer is disclosed.

By adding a cationic polymer (in particular, a copolymer of aminoalkylmethacrylate or polyvinyl acetal diethylaminoacetate) or a cationic polymer plus a viscous polymer to a medicine of high molecular weight for producing a preparation in powder form, it is possible to achieve effective absorption of the medicine of high molecular weight through mucosa.

SEQUENCE LISTING

<110> KIRIN-AMGEN INC.

<120> High molecular weight medicine-containing preparation in
powder form for administration through mucosa

<130>

<140> PCT/JP99/03563
<141> 1999-07-01

<150> JP 10-192722
<151> 1998-07-08

<150> JP 11-81549
<151> 1999-03-25

<160> 3

<210> 1
<211> 175
<212> PRT

<400> 1
Met Thr Pro Leu GlY Pro Ala Ser Ser Leu Pro Gln Ser Phe Leu Leu
 -1  +1              5                  10                  15
Lys Cys Leu Glu Gln Val Arg Lys Ile Gln Gly Asp Gly Ala Ala Leu

20 25 30
Gln Glu Lys Leu Cys Ala Thr Tyr Lys Leu Cys His Pro Glu Glu Leu
35 40 45
Val Leu Leu Gly His Ser Leu Gly Ile Pro Trp Ala Pro Leu Ser Ser
50 55 60
Cys Pro Ser Gln Ala Leu Gln Leu Ala Gly Cys Leu Ser Gln Leu His
65 70 75
Ser Gly Leu Phe Leu Tyr Gln Gly Leu Leu Gln Ala Leu Glu Gly Ile
80 85 90 95
Ser Pro Glu Leu Gly Pro Thr Leu Asp Thr Leu Gln Leu Asp Val Ala
100 105 110
Asp Phe Ala Thr Thr Ile Trp Gln Gln Met Glu Glu Leu Gly Met Ala
115 120 125
Pro Ala Leu Gln Pro Thr Gln Gly Ala Met Pro Ala Phe Ala Ser Ala
130 135 140
Phe Gln Arg Arg Ala Gly Gly Val Leu Val Ala Ser His Leu Gln Ser
145 150 155
Phe Leu Glu Val Ser Tyr Arg Val Leu Arg His Leu Ala Gln Pro
160 165 170

<210> 2
<211> 174
<212> PRT
<213> Homo sapiens

<400> 2
Thr Pro Leu Gly Pro Ala Ser Ser Leu Pro Gln Ser Phe Leu Leu Lys
1               5                   10                  15

Cys Leu Glu Gln Val Arg Lys Ile Gln Gly Asp Gly Ala Ala Leu Gln
            20                  25                  30
Glu Lys Leu Cys Ala Thr Tyr Lys Leu Cys His Pro Glu Glu Leu Val
        35                  40                  45
Leu Leu Gly His Ser Leu Gly Ile Pro Trp Ala Pro Leu Ser Ser Cys
    50                  55                  60
Pro Ser Gln Ala Leu Gln Leu Ala Gly Cys Leu Ser Gln Leu His Ser
65                  70                  75                  80
Gly Leu Phe Leu Tyr Gln Gly Leu Leu Gln Ala Leu Glu Gly Ile Ser
                85                  90                  95
Pro Glu Leu Gly Pro Thr Leu Asp Thr Leu Gln Leu Asp Val Ala Asp
            100                 105                 110
Phe Ala Thr Thr Ile Trp Gln Gln Met Glu Glu Leu Gly Met Ala Pro
        115                 120                 125
Ala Leu Gln Pro Thr Gln Gly Ala Met Pro Ala Phe Ala Ser Ala Phe
    130                 135                 140
Gln Arg Arg Ala Gly Gly Val Leu Val Ala Ser His Leu Gln Ser Phe
145                 150                 155                 160
Leu Glu Val Ser Tyr Arg Val Leu Arg His Leu Ala Gln Pro
                165                 170

<210> 3
<211> 175
<212> PRT

<400> 3
Met Ala Pro Thr Tyr Arg Ala Ser Ser Leu Pro Gln Ser Phe Leu Leu
-1 +1 5 10 15

Lys Ser Leu Glu Gln Val Arg Lys Ile Gln Gly Asp Gly Ala Ala Leu
20 25 30
Gln Glu Lys Leu Cys Ala Thr Tyr Lys Leu Cys His Pro Glu Glu Leu
35 40 45
Val Leu Leu Gly His Ser Leu Gly Ile Pro Trp Ala Pro Leu Ser Ser
50 55 60
Cys Pro Ser Gln Ala Leu Gln Leu Ala Gly Cys Leu Ser Gln Leu His
65 70 75
Ser Gly Leu Phe Leu Tyr Gln Gly Leu Leu Gln Ala Leu Glu Gly Ile
80 85 90 95
Ser Pro Glu Leu Gly Pro Thr Leu Asp Thr Leu Gln Leu Asp Val Ala
100 105 110
Asp Phe Ala Thr Thr Ile Trp Gln Gln Met Glu Glu Leu Gly Met Ala
115 120 125
Pro Ala Leu Gln Pro Thr Gln Gly Ala Met Pro Ala Phe Ala Ser Ala
130 135 140
Phe Gln Arg Arg Ala Gly Gly Val Leu Val Ala Ser His Leu Gln Ser
145 150 155
Phe Leu Glu Val Ser Tyr Arg Val Leu Arg His Leu Ala Gln Pro
160 165 170

SEQUENCE LISTING

<110> KIRIN-AMGEN INC.

<120> High molecular weight medicine-containing preparation in powder form for
administration through mucosa

<130> EP05025878

<140> PCT/JP99/03563
<141> 1999-07-01

<150> JP 10-192722
<151> 1998-07-08

<150> JP 11-81549
<151> 1999-03-25

<160> 3

<210> 1
<211> 175
<212> PRT
<213> Artificial Sequence

<400> 1
   Met Thr Pro Leu GlY Pro Ala Ser Ser Leu Pro Gln Ser Phe Leu Leu
   -1 +1 5 10 15
   Lys Cys Leu Glu Gln Val Arg Lys Ile Gln Gly Asp Gly Ala Ala Leu
   20 25 30
   Gln Glu Lys Leu Cys Ala Thr Tyr Lys Leu Cys His Pro Glu Glu Leu
   35 40 45
   Val Leu Leu Gly His Ser Leu Gly Ile Pro Trp Ala Pro Leu Ser Ser
   50 55 60
   Cys Pro Ser Gln Ala Leu Gln Leu Ala Gly Cys Leu Ser Gln Leu His
   65 70 75
   Ser Gly Leu Phe Leu Tyr Gln Gly Leu Leu Gln Ala Leu Glu Gly Ile
   80 85 90 95
   Ser Pro Glu Leu Gly Pro Thr Leu Asp Thr Leu Gln Leu Asp Val Ala
                   100                 105                 110
   Asp Phe Ala Thr Thr Ile Trp Gln Gln Met Glu Glu Leu Gly Met Ala
               115                 120                 125
   Pro Ala Leu Gln Pro Thr Gln Gly Ala Met Pro Ala Phe Ala Ser Ala
           130                 135                 140
   Phe Gln Arg Arg Ala Gly Gly Val Leu Val Ala Ser His Leu Gln Ser
       145                 150                 155
   Phe Leu Glu Val Ser Tyr Arg Val Leu Arg His Leu Ala Gln Pro
   160                 165                 170

<210> 2
<211> 174
<212> PRT
<213> Homo sapiens

<400> 2
Thr Pro Leu Gly Pro Ala Ser Ser Leu Pro Gln Ser Phe Leu Leu Lys
  1                   5                             10                            15
Cys Leu Glu Gln Val Arg Lys Ile Gln Gly Asp Gly Ala Ala Leu Gln
                20                            25                            30
Glu Lys Leu Cys Ala Thr Tyr Lys Leu Cys His Pro Glu Glu Leu Val
        35                            40                            45
Leu Leu Gly His Ser Leu Gly Ile Pro Trp Ala Pro Leu Ser Ser Cys
    50                            55                            60
Pro Ser Gln Ala Leu Gln Leu Ala Gly Cys Leu Ser Gln Leu His Ser
65                            70                            75                            80
Gly Leu Phe Leu Tyr Gln Gly Leu Leu Gln Ala Leu Glu Gly Ile Ser
                        85                            90                            95
Pro Glu Leu Gly Pro Thr Leu Asp Thr Leu Gln Leu Asp Val Ala Asp
                100                           105                           110
Phe Ala Thr Thr Ile Trp Gln Gln Met Glu Glu Leu Gly Met Ala Pro
        115                           120                           125
Ala Leu Gln Pro Thr Gln Gly Ala Met Pro Ala Phe Ala Ser Ala Phe
    130                           135                           140
Gln Arg Arg Ala Gly Gly Val Leu Val Ala Ser His Leu Gln Ser Phe
145                           150                           155                           160
Leu Glu Val Ser Tyr Arg Val Leu Arg His Leu Ala Gln Pro
                        165                           170

<210> 3

<211> 175

<212> PRT

<213> Artificial Sequence

<400> 3

Met Ala Pro Thr Tyr Arg Ala Ser Ser Leu Pro Gln Ser Phe Leu Leu
 -1  +1               5                  10                  15

Lys Ser Leu Glu Gln Val Arg Lys Ile Gln Gly Asp Gly Ala Ala Leu
                 20                  25                  30

Gln Glu Lys Leu Cys Ala Thr Tyr Lys Leu Cys His Pro Glu Glu Leu
             35                  40                  45

Val Leu Leu Gly His Ser Leu Gly Ile Pro Trp Ala Pro Leu Ser Ser
         50                  55                  60

Cys Pro Ser Gln Ala Leu Gln Leu Ala Gly Cys Leu Ser Gln Leu His
     65                  70                  75

Ser Gly Leu Phe Leu Tyr Gln Gly Leu Leu Gln Ala Leu Glu Gly Ile
 80                  85                  90                  95

Ser Pro Glu Leu Gly Pro Thr Leu Asp Thr Leu Gln Leu Asp Val Ala
                100                 105                 110

Asp Phe Ala Thr Thr Ile Trp Gln Gln Met Glu Glu Leu Gly Met Ala
            115                 120                 125

Pro Ala Leu Gln Pro Thr Gln Gly Ala Met Pro Ala Phe Ala Ser Ala
        130                 135                 140

Phe Gln Arg Arg Ala Gly Gly Val Leu Val Ala Ser His Leu Gln Ser
    145                 150                 155

Phe Leu Glu Val Ser Tyr Arg Val Leu Arg His Leu Ala Gln Pro
160                 165                 170

## Description

### Technical Field of the Invention

The present invention relates to a preparation for administration through mucosa, containing a medicine of high molecular weight as an active ingredient. More particularly, the invention relates to a preparation in powder form for administration through mucosa, comprising a medicine of high molecular weight and a cationic polymer. In particular, the invention relates to a preparation in a powder form for administration through nasal mucosa.

### Background Art

Currently, high molecular weight medicines are administered to patients by intravenous or subcutaneous injection. However, since the administration by injection is difficult to be performed by patients themselves and is accompanied with pain, administration through mucosa is desired as a simpler method than injection. Specific examples of administration through mucosa include administration through nasal mucosa, ocular mucosa, oral mucosa, pulmonary mucosa or vaginal mucosa; or through the mucosa of digestive tract such as gastric mucosa, small intestinal mucosa, large intestinal mucosa or rectal mucosa. Among all, administration through nasal mucosa is attracting attention as a relatively simple administration method by which rapid absorption of medicines and positive effect can be achieved. However, the absorbability depends on the molecular weight of the medicine used. Although medicines with a molecular weight of 1,000 or less are absorbed relatively effectively, effective absorption of those medicines of larger molecular weights is difficult to achieve without some contrivance (C. McMartin et al., J. Pharm. Sci., 76 (7):535-540 (1987)). Thus, it has been difficult to achieve therapeutic effect by administration of high molecular weight medicines through nasal mucosa.

Means to improve the low absorbability of high molecular weight medicines include, methods in which a surfactant or a salt of bile acid is jointly used as an absorption promoting agent (S. Hirai et al., Int. J. Pharm., 9:173-184 (1981); Y. Maitani et al., Drug Design and Delivery, 1:65-70 (1986)); and methods in which cyclodextrin is jointly used as an absorption promoting agent (N.G.M. Schipper et al., J. Control Release, 21 (1):173-185 (1992); T. Irie et al., J. Inter. Pharm., 84:129-139 (1992)). However, it is apprehended that these absorption promoting agents may be harmful to nasal mucosa. Also known are methods in which a high molecular weight substance such as albumin, dextran or sodium hyaluronate is jointly used as an absorption promoting agent (T. Igawa et al., Chem. Pharm. Bull. 36(8):3055-3059 (1988); Japanese Unexamined Patent Publication No. 6-65090; Japanese Unexamined Patent Publication No. 8-198772). However, these methods still cannot achieve sufficient absorption promoting effect and have difficulty in industrial production of such compositions. Thus, none of the above-mentioned methods has been put to practical use.

Japanese Unexamined Patent Publication No. 10-95738 discloses a preparation using fluorescein thiocyanate dextran (hereinafter referred to as "FITC-dextran"; molecular weight: 4,400) as,a model drug (substance) of low absorbability. This preparation was obtained by adding FITC-dextran to physiological saline in which arginine, poly-arginine or a salt of poly-arginine was dissolved. When this preparation was administered to the nasal cavity mucosa of Wistar rats, higher FITC-dextran levels in blood were retained.

Japanese Unexamined Patent Publication No. 4-503508 discloses the administration into rat's nostrils of a preparation obtained by adding DEAE-dextran or chitosan to an insulin solution.

Although various methods as described above have been developed, a more effective and practical method is still required as a means to improve the low absorbability of high molecular weight medicines.

Under such circumstances, it is an object of the present invention to provide a preparation for administration through mucosa, in particular through nasal mucosa, which enables safe and effective absorption of a high molecular weight medicine through mucosa. It is another object of the invention to provide a pharmaceutical composition in powder form which enables safe and effective absorption of a high molecular weight medicine by living bodies.

### Disclosure of the Invention

As a result of intensive and extensive researches toward the development of those preparations which enable safe and effective absorption of a high molecular weight medicine through mucosa, the present inventors have found 1) that a cationic polymer promotes the absorption of a high molecular weight medicine through mucosa by expanding tight junctions of mucosal tissues; and 2) that combined use of a cationic polymer with a viscous polymer further enhances the absorption since the viscous polymer extends the residence time of the relevant preparation in mucosa. Thus, the present invention has been achieved. Also, the present inventors have found that, among cationic polymers, a copolymer of aminoalkylmethacrylate or polyvinyl acetal diethylaminoacetate is superior to poly-L-arginine (which is also a cationic polymer) in absorption promoting effect.

The present invention provides a preparation in powder form for administration through mucosa, in particular for pernasal administration, comprising a medicine of high molecular weight and a cationic polymer. It is preferred that the powder form preparation of the invention for administration through mucosa further comprise a viscous polymer. Specific examples of cationic polymers include copolymers of aminoalkylmethacrylate, polyvinyl acetal diethylaminoacetate and poly-L-arginine. Copolymers of aminoalkylmethacrylate and polyvinyl acetal diethylaminoacetate are preferable. As a viscous polymer, hydroxypropylmethyl cellulose may be mentioned. A medicine of high molecular weight may be selected from the group consisting of bioactive peptides and proteins, antibodies, vaccines, and antigens. The preparation of the invention is especially effective for the administration of granulocyte colony-stimulating factor, insulin, erythropoietin, growth hormone or influenza antigens through mucosa, in particular through nasal mucosa.

The present invention also provides a pharmaceutical composition in powder form, comprising a medicine of high molecular weight and a cationic polymer. In the pharmaceutical composition of the invention in powder form, a medicine of high molecular weight may be selected from the group consisting of bioactive peptides and proteins, antibodies, vaccines, and antigens. The pharmaceutical composition of the invention in powder form is especially effective for the administration of granulocyte colony-stimulating factor, insulin, erythropoietin, growth hormone or influenza antigens.

Hereinbelow, the present invention will be described in detail.

In one embodiment of the invention, the powder form preparation of the invention for administration through mucosa is obtained by adding to a medicine of high molecular weight an excipient (e.g. saccharides) and a cationic polymer and optionally a viscous polymer and, if necessary, appropriate additives and then freeze-drying or spray-drying the resultant mixture. "A medicine of high molecular weight" used in the invention refers to a bioactive peptide or protein; antibody, vaccine, antigen or the like. Specific examples include the following substances, which are not intended to limit the present invention: calcitonin, insulin, proinsulin, vasopressin, desmopressin, luteinizing hormone, luteinizing hormone-releasing hormone, somatostatin, prolactin, glucagon, gastrin, secretin, kallikrein, urokinase, neurotensin, enkephalin, kyotorphin, endorphin, endothelin, angiotensin, transferrin, atrial natriuretic polypeptide, epithelial cell growth factor, growth hormone, parathyroid hormone, interferons, interleukins, tumor necrosis factor, leukemia inhibitory factor, hematopoietic stem cell growth factor, erythropoietin, granulocyte colony-stimulating factor (G-CSF), granulocyte macrophage-stimulating factor, macrophage colony-stimulating factor, thrombopoietin, superoxide dismutase, tissue plasminogen activator, antithrombin, blood coagulation factors, anti-IgE antibodies, anti-IgA antibodies, anti-tumor antibodies, antibodies to tumor necrosis factor, anti-interleukin antibodies, HIV-neutralizing antibodies, anti-platelet antibodies, anti-hepatitis virus antibodies, hepatitis vaccines, influenza vaccines (influenza antigens), pertussis vaccine, diphtheria vaccine, tetanus toxoids vaccine, peptides or proteins such as pollen from Japanese cedar or ragweed which may act as antigen, such peptides or proteins conjugated to haptens, and mixtures of such peptides, proteins or conjugates with adjuvants. It is easily presumed that the present invention will also improve the absorbability through mucosa, in particular through nasal mucosa, of medicines which have smaller molecular weights than the above enumerated high molecular weight medicines. Thus, it is believed that the application of the present invention to them will be also useful.

Examples of G-CSF which is one of the high molecular weight medicines that can be used in the present invention include a polypeptide with human G-CSF activity represented by the amino acid sequence of SEQ ID NO: 1, 2 or 3; and a glycoprotein composed of the above polypeptide and sugar chains added thereto. Further, G-CSF derivatives with G-CSF activity represented by the above-mentioned amino acid sequence which is partially modified (i.e. has substitution, deletion, insertion and/or addition) are also included in the G-CSF of the invention.

These G-CSFs may be extracted/separated/purified from natural products, or they may be produced by transformants obtained by recombinant techniques and then isolated/purified. Examples of host cells for such transformation include E. coli and mammal cells (e.g. C127, CHO cells). Detailed methods for producing these G-CSFs are disclosed, for example, in Japanese Unexamined Patent Publication/PCT No. 63-500636 and Japanese Unexamined Patent Publication Nos. 62-236497, 62-236488 and 63-267292.

The content of a medicine of high molecular weight in the powder form preparation of the invention is usually 0.01 to 90% (w/w), preferably 0.1 to 50% (w/w).

"A cationic polymer" used in the invention refers to a polymer which has a cation charge in its monomer units forming a repetitive structure, or a polymer which has such a structure that it acquires a cation charge upon dissolution. The cationic polymer used in the invention may be any cationic polymer as long as it promotes the absorption of high molecular weight medicines through mucosa. Specifically, a copolymer of aminoalkylmethacrylate, polyvinyl acetal diethylaminoacetate, poly-L-arginine or the like may be used. A copolymer of aminoalkylmethacrylate is available from, for example, Rohm Pharma under the trade name Eudragit E or Eudragit RS. Eudragit E is a copolymer of methyl methacrylate, butyl methacrylate and dimethylaminoethyl methacrylate with an average molecular weight of 150,000. Polyvinyl acetal diethylaminoacetate is available from, for example, Sankyo Co., Ltd. under the trade name AEA. This is a polymer with an average molecular weight of 65,000 which is obtained by dehydrating polyvinyl alcohol and acetaldehyde to generate acetal and hydroxyl, and then attaching diethyl aminoacetate to a part of the acetal and hydroxyl by ester linkage. Poly-L-arginine is a polymer of L-arginine. Its average molecular weight is 1000 to 1,000,000. Preferably, this polymer has an average molecular weight of 12,100 to 92,000, more preferably 92,000. Poly-L-arginine is available from Sigma. The content of a cationic polymer in the powder form preparation of the invention for administration through mucosa is usually 0.1 to 90% (w/w), preferably 1 to 50% (w/w) .

"A viscous polymer" used in the invention refers to a polymer which becomes viscous when dissolved or swollen. The viscous polymer used in the invention may be any viscous polymer as long as it increases the absorption of a medicine of high molecular weight when used in combination with a cationic polymer, as compared to the case when the cationic polymer is used alone. Specific examples of such viscous polymers include hydroxypropylmethyl cellulose, hydroxypropyl cellulose, carboxyvinyl polymer, agar powder and gum arabic powder. The content of a viscous polymer in the powder form preparation of the invention for administration through mucosa is usually 0.1 to 90% (w/w), preferably 1 to 50% (w/w).

An excipient used in the invention is, typically, a saccharide.
Specific examples of saccharides include xylitol, fructose, sorbitol, lactose, inositol, sucrose and mannitol. Other examples of excipients include starches, inorganic substances, organic acids and amino acids. As starches, corn starch, wheat starch, potato starch and the like may be enumerated. As inorganic substances, calcium phosphate, calcium hydrogenphosphate, disodium hydrogenphosphate, sodium dihydrogenphosphate, magnesium carbonate, sodium chloride, calcium sulfate and the like may be enumerated. As organic substances, succinic acid, tartaric acid, citric acid, fumaric acid, malic acid, gluconic acid, glucuronic acid, salts thereof, and the like may be enumerated. As amino acids, L-arginine, D,L-methionine, L-phenylalanine, L-glutamic acid and the like may be enumerated. The content of the excipient in the powder form preparation of the invention for administration through mucosa is usually 1 to 90% (w/w), preferably 5 to 80% (w/w).

If necessary, additives such as a lubricant may be used in the present invention. Specific examples of lubricants include magnesium stearate, stearic acid and talc. The content of the additives in the powder form preparation of the invention for administration through mucosa is usually 0.01 to 90% (w/w), preferably 0.05 to 50% (w/w).

Hereinbelow, a exemplary method for producing the powder form preparation of the invention for administration through mucosa will be described briefly.

A buffer solution containing G-CSF is mixed with a buffer solution in which a cationic polymer, an excipient such as sucrose or mannitol and, optionally, a viscous polymer have been dissolved in advance. The resultant mixture is spray-dried to obtain a powder.

Necessary amounts of the resultant powder are weighed out and packed in capsules to obtain a preparation in powder form for administration through mucosa.

The thus prepared powder of the preparation for administration through mucosa is usually 0.1 to 500 µm, preferably 5 to 100 µm in particle size.

The powder of the preparation for administration through mucosa is easy to handle when it is packed in capsules. As a material for the capsule base, gelatin, hydroxypropylmethyl cellulose, methyl cellulose, starch or the like may be used. Glycerol, sorbitol, carrageenan, polyethylene glycol, gum arabic or the like may be added to the above material to increase plasticity.

Additionally, potassium chloride, sucrose, a coloring agent and titanium oxide may also be added.

The preparation of the invention in powder form for administration through mucosa may be applied to the mucous membrane of patients at the time of need or at an appropriate frequency. Specific examples of mucosa include nasal mucosa, ocular mucosa, oral mucosa, pulmonary mucosa, vaginal mucosa and mucous membranes of digestive tract such as gastric mucosa, small intestinal mucosa, large intestinal mucosa and rectal mucosa. For example, when the preparation of the invention is administered through nasal mucosa, a capsule containing the powder form preparation is set in a small-sized sprayer (Publizer™). After a hole is made in the capsule, the nozzle of the sprayer is inserted into one of the nostrils of the patient. While he is breathing in through the nose, the patient presses the rubber ball of the sprayer to thereby spray the powder form preparation into the nasal cavity. A preparation of the invention containing granulocyte colony-stimulating factor as an active ingredient may be administered to patients 1 to 4 times a day such that the dose of the active ingredient is 1-500 µ g/kg/day, preferably 5-100µg/kg/day. A preparation of the invention containing insulin as an active ingredient may be administered to patients 1 to 4 times a day such that the dose of the active ingredient is 0.1-100 U/kg/day, preferably 0.5-20 U/kg/day. A preparation of the invention containing erythropoietin as an active ingredient may be administered to patients 1 to 4 times a day such that the dose of the active ingredient is 50-50,000 IU/kg/day, preferably 200-8,000 IU/kg/day. A preparation of the invention containing growth hormone as an active ingredient may be administered to patients 1 to 4 times a day such that the dose of the active ingredient is 0.1-50 IU/kg/day, preferably 0.4-15 IU/kg/day. A preparation of the invention containing an influenza antigen as an active ingredient may be administered to persons in need of such a preparation 1 to 4 times a day with an interval of 2-6 weeks such that the dose of the active ingredient is 0.5-200 CCA/kg/day, preferably 20-40 CCA/kg/day.

The present specification includes the contents of the specifications and the attached drawings of Japanese Patent Application Nos. 10-192722 and 11-81549 based on which the present application claims priority.

### Best Modes for Carrying Out the Invention

Hereinbelow, the present invention will be described specifically with reference to the following Examples. However, the scope of the invention is not limited to these Examples.

The cationic polymers, sucrose, D-mannitol, hydroxypropylmethyl cellulose, sodium hyaluronate and components of buffer solutions dissolving them (buffer components) used in the following Examples and Comparative Examples are as described below. Cationic polymers
Poly-L-arginine (Sigma)
Aminoalkylmethacrylate copolymer E (Rohm Pharma; Trade Name: Eud
ragit E100)
Polyvinyl acetal diethylaminoacetate (Sankyo Co., Ltd.; Trade Na me: AEA)
Diethylaminoethyl (DEAE)-dextran (Fluka)
Chitosan (Chitosan 8B; manufactured by Katokichi Co., Ltd. and s
old by Funakoshi)
Sucrose (Kosakai Pharmaceutical; saccharose prepared according to the Japanese Pharmacopoeia)
D-mannitol (Kao Corp.; Trade Name: Nikkyoku Mannitol Kao)
Hydroxypropylmethyl cellulose (Shin-Etsu Chemical; Trade Name: Metholo se 60SH4000)
Sodium hyaluronate (Tokyo Kasei Organic Chemicals) Buffer components
Citric acid (Oriental Pharmaceutical & Synthetic Chemical)
Phosphoric acid (Kokusan Kagaku)

The medicines of high molecular weight used in the following Examples are as described below.

The granulocyte colony-stimulating factor (G-CSF) used is a polypeptide having the amino acid sequence shown in SEQ ID NO: 1, produced by a transformed E. coli (see Japanese Unexamined Patent Publication/PCT No. 63-500636). The thus obtained G-CSF was concentrated, followed by buffer replacement to prepare a buffer solution ontaining G-CSF.

The insulin used is a commercial product (Boehringer Mannheim; recombinant human insulin; Mw = ca. 5700) .

The erythropoietin used is a commercial product (Kirin Brewery; recombinant human erythropoietin; Mw = ca. 30,000).

The growth hormone used is a commercial product (Chemicon; recombinant human growth hormone; Mw = ca. 22,000).

The influenza A antigen used is a commercial product (Chemicon).

### <EXAMPLE 1>

To a buffer solution containing G-CSF, a buffer solution containing sucrose and poly-L-arginine was added. The resultant mixture was spray-dried to thereby obtain a powder form preparation for pernasal administration having the following formula.

| | |
|---|---|
| G-CSF | 20% (w/w) |
| Poly-L-arginine | 20% (w/w) |
| Sucrose | 26% (w/w) |
| Buffer components | appropriate amounts |
| Total | 100% (w/w) |

### <EXAMPLE 2>

To a buffer solution containing G-CSF, a buffer solution containing sucrose and polyvinyl acetal diethylaminoacetate (AEA) was added. The resultant mixture was spray-dried to thereby obtain a powder form preparation for pernasal administration having he following formula.

| | |
|---|---|
| G-CSF | 20% (w/w) |
| AEA | 20% (w/w) |
| Sucrose | 26% (w/w) |
| Buffer components | appropriate amounts |
| Total | 100% (w/w) |

### <EXAMPLE 3>

To a buffer solution containing G-CSF, a buffer solution containing sucrose and aminoalkylmethacrylate copolymer E (Eudragit E100) was added. The resultant mixture was spray-dried to thereby obtain a powder form preparation for pernasal administration having the following formula.

| | |
|---|---|
| G-CSF | 20% (w/w) |
| Eudragit E100 | 20% (w/w) |
| Sucrose | 26% (w/w) |
| Buffer components | appropriate amounts |
| Total | 100% (w/w) |

### <EXAMPLE 4>

To a buffer solution containing G-CSF, a buffer solution containing sucrose and aminoalkylmethacrylate copolymer E (Eudragit E100) was added. The resultant mixture was spray-dried to thereby obtain a powder form preparation for pernasal administration having the following formula.

| | |
|---|---|
| G-CSF | 20% (w/w) |
| Eudragit E100 | 10% (w/w) |
| Sucrose | 63% (w/w) |
| Buffer components | appropriate amounts |
| Total | 100% (w/w) |

### <EXAMPLE 5>

To a buffer solution containing G-CSF, a buffer solution containing sucrose and aminoalkylmethacrylate copolymer E (Eudragit E100) was added. The resultant mixture was spray-dried to thereby obtain a powder form preparation for pernasal administration having the following formula.

| | |
|---|---|
| G-CSF | 20% (w/w) |
| Eudragit E100 | 20% (w/w) |
| Sucrose | 53% (w/w) |
| Buffer components | appropriate amounts |
| Total | 100% (w/w) |

### <EXAMPLE 6>

To a buffer solution containing G-CSF, a buffer solution containing sucrose and aminoalkylmethacrylate copolymer E (Eudragit E100) was added. The resultant mixture was spray-dried to thereby obtain a powder form preparation for pernasal administration having the following formula.

| | |
|---|---|
| G-CSF | 20% (w/w) |
| Eudragit E100 | 30% (w/w) |
| Sucrose | 43% (w/w) |
| Buffer components | appropriate amounts |
| Total | 100% (w/w) |

### <EXAMPLE 7>

To a buffer solution containing G-CSF, a buffer solution containing sucrose and aminoalkylmethacrylate copolymer E (Eudragit E100) was added. The resultant mixture was spray-dried to thereby obtain a powder form preparation for pernasal administration having the following formula.

| | |
|---|---|
| G-CSF | 20% (w/w) |
| Eudragit E100 | 57% (w/w) |
| Buffer components | appropriate amounts |
| Total | 100% (w/w) |

### <EXAMPLE 8>

To a buffer solution containing G-CSF, a buffer solution containing sucrose and aminoalkylmethacrylate copolymer E (Eudragit E100) was added. The resultant mixture was spray-dried to thereby obtain a powder form preparation for pernasal administration having the following formula.

| | |
|---|---|
| G-CSF | 20% (w/w) |
| Eudragit E100 | 20% (w/w) |
| Sucrose | 47% (w/w) |
| Buffer components | appropriate amounts |
| Total | 100% (w/w) |

### <EXAMPLE 9>

To a buffer solution containing G-CSF, a buffer solution containing sucrose, aminoalkylmethacrylate copolymer E (Eudragit E100) and hydroxypropylmethyl cellulose (HPMC) was added. The resultant mixture was spray-dried to thereby obtain a powder form preparation for pernasal administration having the following formula.

| | |
|---|---|
| G-CSF | 20% (w/w) |
| Eudragit E100 | 20% (w/w) |
| HPMC | 10% (w/w) |
| Sucrose | 37% (w/w) |
| Buffer components | appropriate amounts |
| Total | 100% (w/w) |

### <EXAMPLE 10>

To a buffer solution containing G-CSF, a buffer solution containing sucrose, aminoalkylmethacrylate copolymer E (Eudragit E100) and hydroxypropylmethyl cellulose (HPMC) was added. The resultant mixture was spray-dried to thereby obtain a powder form preparation for pernasal administration having the following formula.

| | |
|---|---|
| G-CSF | 20% (w/w) |
| Eudragit E100 | 20% (w/w) |
| HPMC | 20% (w/w) |
| Sucrose | 27% (w/w) |
| Buffer components | appropriate amounts |
| Total | 100% (w/w) |

### <COMPARATIVE EXAMPLE 1>

To a buffer solution containing G-CSF, a buffer solution containing sucrose was added. The resultant mixture was spray-dried to thereby obtain a powder form preparation for pernasal administration having the following formula.

| | |
|---|---|
| G-CSF | 20% (w/w) |
| Sucrose | 46% (w/w) |
| Buffer components | appropriate amounts |
| Total | 100% (w/w) |

### <COMPARATIVE EXAMPLE 2>

To a buffer solution containing G-CSF, a buffer solution containing sucrose and sodium hyaluronate was added. The resultant mixture was spray-dried to thereby obtain a powder form preparation for pernasal administration having the following formula.

| | |
|---|---|
| G-CSF | 20% (w/w) |
| Sodium hyaluronate | 20% (w/w) |
| Sucrose | 26% (w/w) |
| Buffer components | appropriate amounts |
| Total | 100% (w/w) |

### <EXPERIMENTAL EXAMPLE 1>

Male beagles were used in this experiment. The preparations from Example 1 and Comparative Example 1 were separately packed in gelatin capsules such that each capsule would give the dog 100 0 µg of G-CSF per kg of body weight. The gelatin capsule was mounted in Publizer™ (Ishikawa Seisakusho) having a silicone tube about 2.5 cm in length bonded to its tip. Then, the silicone tube portion was inserted into the nasal cavity of the dog through one of its nostrils, followed by pressing the rubber ball portion of the Publizer™ to administer the preparation. After the administration, blood samples were taken from the forearm vein at regular intervals. Blood G-CSF levels were determined by ELISA (T. Ichikawa et al., Experimental Hematology 23: 192-195 (1955)). Table 1 shows the value of area under the blood G-CSF level vs time curve (AUC_{G}) for each preparation. The preparation of Example 1 containing poly-L-arginine as a cation polymer exhibited a higher AUC_{G} value than the preparation of Comparative Example 1 containing no high molecular weight substances. Thus, it was found that the addition of poly-L-arginine promotes the absorption of G-CSF through nasal mucosa.

**Table 1.**

| Preparation administered | Comparative Ex.1 | Example 1 |
|---|---|---|
| AUC_{G} 0 → 32 hr ( · hr · ml⁻¹) | 5.3 | 10.3 |

### <EXPERIMENTAL EXAMPLE 2>

Male beagles were used in this experiment. The preparations from Comparative Examples 1 and 2 were separately packed in gelatin capsules such that each capsule would give the dog 100 µg of G-CSF per kg of body weight. The gelatin capsule was mounted in Publizer™ (Ishikawa Seisakusho) having a silicone tube about 2.5 cm in length bonded to its tip. Then, the silicone tube portion was inserted into the nasal cavity of each dog through one of the nostrils, followed by pressing the rubber ball portion of the Publizer™ to administer the preparation. After the administration, blood samples were taken from the forearm vein at regular intervals. Blood G-CSF levels were determined by ELISA. Table 2 shows the value of area under the blood G-CSF level vs time curve (AUC_{G} ) for each preparation. The preparation of Comparative Example 2 containing sodium hyaluronate (a non-cationic polymer) exhibited substantially the same AUC_{G} value as the preparation of Comparative Example 1 containing no high molecular weight substances. Thus, the addition of sodium hyaluronate showed little promotive effect on G-CSF absorption.

**Table 2.**

| Preparation administered | Comparative Ex. 1 | Comparative Ex. 2 |
|---|---|---|
| AUC_{G} 0 → 32 hr (ng · hr · ml⁻¹) | 4.8 | 4.6 |

### <EXPERIMENTAL EXAMPLE 3>

Male beagles were used in this experiment. The preparations from Examples 1, 2 and 3 were separately packed in gelatin capsules such that each capsule would give the dog 100 µg of G-CSF per kg of body weight. The gelatin capsule was mounted in Publizer™ (Ishikawa Seisakusho) having a silicone tube about 5.0 cm in length bonded to its tip. Then, the silicone tube portion was inserted into the nasal cavity of each dog through one of the nostrils, followed by pressing the rubber ball portion of the Publizer™ to administer the preparation. After the administration, blood samples were taken from the forearm vein at regular intervals. Leukocyte counts in blood samples were determined with a micro-cell counter. Blood G-CSF levels were determined by ELISA. Table 3 shows the values of area under the increased leukocyte count vs time curve (AUC_{W}) and of area under the blood G-CSF level vs time curve (AUC_{G}) for the preparations tested. As a result, it was found that AEA and Eudragit E100 have a better absorption-promoting effect than poly-L-arginine.

**Table 3.**

| Preparation administered | Example 1 | Example 2 | Example 3 |
|---|---|---|---|
| AUC_{W} 0 → 72 hr (count · hr · ml⁻¹) | 3083 | 5095 | 5707 |
| AUC_{G} 0 → 31 hr (ng · hr · ml⁻¹) | 13.4 | 73.0 | 44.4 |

### <EXPERIMENTAL EXAMPLE 4>

Male beagles were used in this experiment. The preparations from Examples 4, 5, 6 and 7 were separately packed in gelatin capsules such that each capsule would give the dog 100 µg of G-CSF per kg of body weight. The gelatin capsule was mounted in Publizer™ (Ishikawa Seisakusho) having a silicone tube about 5.0 cm in length bonded to its tip. Then, the silicone tube portion was inserted into the nasal cavity of each dog through one of the nostrils, followed by pressing the rubber ball portion of the Publizer™ to administer the preparation. After the administration, blood samples were taken from the forearm vein at regular intervals. Leukocyte counts in blood samples were determined with a micro-cell counter. Blood G-CSF levels were determined by ELISA. Table 4 shows the values of area under the increased leukocyte count vs time curve (AUC_{W}) and of area under the blood G-CSF level vs time curve (AUC_{G}) for the preparations tested. The effect of Eudragit E100 was retained in spite of various changes in its content.

**Table 4.**

| Preparation Administered | Example 4 | Example 5 | Example 6 | Example 7 |
|---|---|---|---|---|
| AUC_{W} 0 → 72 hr (count · hr · ml⁻¹) | 3475 | 4053 | 4138 | 4562 |
| AUC_{G} 0 → 31 hr (ng · hr · ml⁻¹) | 25.6 | 27.1 | 16.8 | 11.1 |

### <EXPERIMENTAL EXAMPLE 5>

Male beagles were used in this experiment. The preparations from Examples 8, 9 and 10 were separately packed in gelatin capsules such that each capsule would give the dog 100µg of G-CSF per kg of body weight.
The gelatine capsule was mounted in Publizer™ (Ishikawa Seisakusho) having a silicone tube about 5.0 cm in length bonded to its tip. Then, the silicone tube portion was inserted into the nasal cavity of each dog through one of the nostrils, followed by pressing the rubber ball portion of the Publizer™ to administer the preparation. After the administration, blood samples were taken from the forearm vein at regular intervals. Leukocyte counts in blood samples were determined with a micro-cell counter. Blood G-CSF levels were determined by ELISA. Table 4 shows the values of area under the increased leukocyte count vs time curve (AUC_{W}) and of area under the blood G-CSF level vs time curve (AUC_{G}) for the preparations tested. As a result, it became evident that the addition of HPMC together with Eudragit E100 enhances the absorption promoting effect as compared to the addition of Eudragit alone.

**Table 5.**

| Preparation administered | Example 8 | Example 9 | Example 10 |
|---|---|---|---|
| AUC_{W} 0 → 72 hr . (count · hr · ml⁻¹) | 3988 | 5482 | 5618 |
| AUC_{G} 0 → 31 hr (ng · hr · ml⁻¹) | 19.9 | 54.5 | 76.7 |

### <EXAMPLE 11>

To a buffer solution containing G-CSF, a buffer solution containing D-mannitol and aminoalkylmethacrylate copolymer E (Eudragit E100) was added. The resultant mixture was spray-dried to thereby obtain a powder form preparation for pernasal administration having the following formula.

| | |
|---|---|
| G-CSF | 10.0% (w/w) |
| Eudragit E100 | 7.5% (w/w) |
| D-mannitol | 75.0% (w/w) |
| Buffer components | appropriate amounts |
| Total | 100 % (w/w) |

### <COMPARATIVE EXAMPLE 3>

To a buffer solution containing G-CSF, a buffer solution containing D-mannitol was added. The resultant mixture was spray-dried to thereby obtain a powder form preparation for pernasal administration having the following formula.

| | |
|---|---|
| G-CSF | 10.0% (w/w) |
| D-mannitol | 81.8% (w/w) |
| Buffer components | appropriate amounts |
| Total | 100 % (w/w) |

### <EXPERIMENTAL EXAMPLE 6>

Male beagles were used in this experiment. The preparations from Example 11 and Comparative Example 3 were separately packed in gelatin capsules such that each capsule would give the dog 50 µg of G-CSF per kg of body weight. The gelatin capsule was mounted in Publizer™ (Ishikawa Seisakusho) having a silicone tube about 5.0 cm in length bonded to its tip. Then, the silicone tube portion was inserted into the nasal cavity of each dog through one of the nostrils, followed by pressing the rubber ball portion of the Publizer™ to administer the preparation. After the administration, blood samples were taken from the forearm vein at regular intervals. Blood G-CSF levels were determined by ELISA. Table 6 shows the values of area under the blood G-CSF level us time curve (AUC_{G}) for the preparations tested. As a result, it was found that Eudragit E100 remarkably promotes the adsorption of G-CSF through nasal mucosa.

**Table 6.**

| Preparation administered | Example 11 | Comparative Ex. 3 |
|---|---|---|
| AUC_{G} 0 → 31 hr (ng · hr · ml⁻¹) | 67.1 | 16.4 |

### <EXAMPLE 12>

To a buffer solution containing insulin, a buffer solution containing sucrose, aminoalkylmethacrylate copolymer E (Eudragit E100) and hydroxypropylmethyl cellulose (HPMC) was added. The resultant mixture was spray-dried to thereby obtain a powder form preparation for pernasal administration having the following formula.

| | |
|---|---|
| Insulin | 18% (w/w) |
| Eudragit E100 | 27% (w/w) |
| HPMC | 9% (w/w) |
| Sucrose | 32% (w/w) |
| Buffer components | appropriate amounts |
| Total | 100% (w/w) |

### <EXAMPLE 13>

To a buffer solution containing insulin, a buffer solution containing sucrose, poly-L-arginine and hydroxypropylmethyl cellulose (HPMC) was added. The resultant mixture was spray-dried to thereby obtain a powder form preparation for pernasal administration having the following formula.

| | |
|---|---|
| Insulin | 18% (w/w) |
| Poly-L-arginine | 27% (w/w) |
| HPMC | 9% (w/w) |
| Sucrose | 32% (w/w) |
| Buffer components | appropriate amounts |
| Total | 100% (w/w) |

### <EXAMPLE 14>

To a buffer solution containing insulin, a buffer solution containing sucrose, diethylaminoethyl (DEAE) - dextran and hydroxypropylmethyl cellulose (HPMC) was added. The resultant mixture was spray-dried to thereby obtain a powder form preparation for pernasal administration having the following formula.

| | |
|---|---|
| Insulin | 18% (w/w) |
| DEAE-dextran | 27% (w/w) |
| HPMC | 9% (w/w) |
| Sucrose | 32% (w/w) |
| Buffer components | appropriate amounts |
| Total | 100% (w/w) |

### <EXAMPLE 15>

To a buffer solution containing insulin, a buffer solution containing sucrose, chitosan and hydroxypropylmethyl cellulose (HPMC) was added. The resultant mixture was spray-dried to thereby obtain a powder form preparation for pernasal administration having the following formula.

| | |
|---|---|
| Insulin | 18% (w/w) |
| Chitosan | 27% (w/w) |
| HPMC | 9% (w/w) |
| Sucrose | 32% (w/w) |
| Buffer components | appropriate amounts |
| Total | 100% (w/w) |

### <COMPARATIVE EXAMPLE 4>

To a buffer solution containing insulin, a buffer solution containing sucrose and hydroxypropylmethyl cellulose (HPMC) was added. The resultant mixture was spray-dried to thereby obtain a powder form preparation for pernasal administration having the following formula.

| | |
|---|---|
| Insulin | 18% (w/w) |
| HPMC | 9% (w/w) |
| Sucrose | 60% (w/w) |
| Buffer components | appropriate amounts |
| Total | 100% (w/w) |

### <COMPARATIVE EXAMPLE 5>

The insulin (as described above) was dissolved in a buffer solution to prepare a liquid preparation for subcutaneous administration having the following concentration.

| | |
|---|---|
| Insulin | 1.0 mg/ml |

### <EXPERIMENTAL EXAMPLE 7>

The preparations from Examples 12-15 and Comparative Example 4 were administered to male beagles through the nose; and the preparation from Comparative Example 5 was administered to male beagles subcutaneously. For the pernasal administration group, individual preparations were packed in gelatin capsules such that each capsule would give the dog 70 µg of insulin per kg of body weight. The gelatine capsule was mounted in Publizer™ (Ishikawa Seisakusho) having a silicone tube about 5.0 cm in length bonded to its tip. Then, the silicone tube portion was inserted into the nasal cavity of each dog through one of the nostrils, followed by pressing the rubber ball portion of the Publizer™ to administer the preparation. In the subcutaneous administration group, the preparation from Comparative Example 5 was administered subcutaneously to the back of each dog such that 25 µg of insulin was administered per kg of body weight. After the administration, blood samples were taken from the forearm vein at regular intervals. Blood insulin levels were determined by ELISA. Table 7 shows the value of area under the blood insulin level vs time curve (AUC) for each preparation. As a result, it was found that Eudragit E100 remarkably promotes the pernasal absorption of insulin. Further, its absorption promoting effect was superior to the effect of poly-L-arginine, DEAE-dextran and chitosan. The bioavailability of the insulin in the preparation of Example 12 was 27% as for the subcutaneous administration of insulin.

**Table 7.**

| Preparation administered | Example 12 | Comparative Example 4 | Comparative Example 5 |
|---|---|---|---|
| AUC 0 → 7 hr (ng · hr · ml⁻¹) | 29.0 | 2.6 | 38.8 |

| Preparation administered | Example 13 | Example 14 | Example 15 |
|---|---|---|---|
| AUC 0 → 7 hr (ng · hr · ml⁻¹) | 2.9 | 2.5 | 23.6 |

### <EXAMPLE 16>

To a buffer solution containing G-CSF, a buffer solution containing D-mannitol and aminoalkylmethacrylate copolymer E (Eudragit E100) was added. The resultant mixture was spray-dried to thereby obtain a powder form preparation for pernasal administration having the following formula.

| | |
|---|---|
| G-CSF | 10.0% (w/w) |
| Eudragit E100 | 7.5% (w/w) |
| D-mannitol | 75.2% (w/w) |
| Buffer components | appropriate amounts |
| Total | 100% (w/w) |

### <EXAMPLE 17>

To a buffer solution containing G-CSF, a buffer solution containing D-mannitol and poly-L-arginine was added. The resultant mixture was spray-dried to thereby obtain a powder form preparation for pernasal administration having the following formula.

| | |
|---|---|
| G-CSF | 10.0% (w/w) |
| Poly-L-arginine | 7.5% (w/w) |
| D-mannitol | 75.2% (w/w) |
| Buffer components | appropriate amounts |
| Total | 100% (w/w) |

### <EXAMPLE 18>

To a buffer solution containing G-CSF, a buffer solution containing D-mannitol and diethylaminoethyl (DEAE)-dextran was added. The resultant mixture was spray-dried to thereby obtain a powder form preparation for pernasal administration having the following formula.

| | |
|---|---|
| G-CSF | 10.0% (w/w) |
| DEAE-dextran | 7.5% (w/w) |
| D-mannitol | 75.2% (w/w) |
| Buffer components | appropriate amounts |
| Total | 100% (w/w) |

### <EXAMPLE 19>

To a buffer solution containing G-CSF, a buffer solution containing D-mannitol and chitosan was added. The resultant mixture was spray-dried to thereby obtain a powder form preparation for pernasal administration having the following formula.

| | |
|---|---|
| G-CSF | 10.0% (w/w) |
| Chitosan | 7.5% (w/w) |
| D-mannitol | 75.2% (w/w) |
| Buffer components | appropriate amounts |
| Total | 100 % (w/w) |

### <EXPERIMENTAL EXAMPLE 8>

Male beagles were used in this experiment. The preparations from Examples 16, 17, 18 and 19 were separately packed in gelatin capsules such that each capsule would give the dog 50 µg of G-CSF per kg of body weight. The gelatin capsule was mounted in Publizer™ (Ishikawa Seisakusho) having a silicone tube about 5.0 cm in length bonded to its tip. Then, the silicone tube portion was inserted into the nasal cavity of each dog through one of the nostrils, followed by pressing the rubber ball portion of the Publizer™ to administer the preparation. After the administration, blood samples were taken from the forearm vein at regular intervals. Blood G-CSF levels were determined by ELISA. Table 8 shows the values of area under the blood G-CSF level vs time curve (AUC_{G}) for the preparations tested. As a result, it was found that the powder preparation containing Eudragit E100 exhibits the highest AUC_{G} value. From the above, it became evident that the absorption promoting effect of Eudragit E100 is superior to that of the other polycations, i.e. poly-L-arginine, DEAE-dextran and chitosan.

**Table 8.**

| Preparation administered | Example 16 | Example 17 |
|---|---|---|
| AUC_{G} 0 → 31 hr (ng · hr · ml⁻¹) | 46.4 | 35.7 |

| Preparation administered | Example 18 | Example 19 |
|---|---|---|
| AUC_{G} 0 → 31 hr (ng · hr · ml⁻¹) | 22.9 | 42.8 |

### <EXAMPLE 20>

To a buffer solution containing erythropoietin, a buffer solution containing sucrose, aminoalkylmethacrylate copolymer E (Eudragit E100) and hydroxypropylmethyl cellulose (HPMC) was added. The resultant mixture was spray-dried to thereby obtain a powder form preparation for pernasal administration having the following formula.

| | |
|---|---|
| Erythropoietin | 30% (w/w) |
| Eudragit E100 | 30% (w/w) |
| HPMC | 10% (w/w) |
| Sucrose | 15% (w/w) |
| Buffer components | appropriate amounts |
| Total | 100% (w/w) |

### <COMPARATIVE EXAMPLE 6>

To a buffer solution containing erythropoietin, a buffer solution containing sucrose and hydroxypropylmethyl cellulose (HPMC) was added. The resultant mixture was spray-dried to thereby obtain a powder form preparation for pernasal administration having the following formula.

| | |
|---|---|
| Erythropoietin | 30% (w/w) |
| HPMC | 10% (w/w) |
| Sucrose | 45% (w/w) |
| Buffer components | appropriate amounts |
| Total | 100% (w/w) |

### <EXAMPLE 21>

To a buffer solution containing erythropoietin, a buffer solution containing sucrose, poly-L-arginine and hydroxypropylmethyl cellulose (HPMC) was added. The resultant mixture was spray-dried to thereby obtain a powder form preparation for pernasal administration having the following formula.

| | |
|---|---|
| Erythropoietin | 30% (w/w) |
| Poly-L-arginine | 30% (w/w) |
| HPMC | 10% (w/w) |
| Sucrose | 15% (w/w) |
| Buffer components | appropriate amounts |
| Total | 100% (w/w) |

### <EXAMPLE 22>

To a buffer solution containing erythropoietin, a buffer solution containing sucrose, diethylaminoethyl (DEAE)-dextran and hydroxypropylmethyl cellulose (HPMC) was added. The resultant mixture was spray-dried to thereby obtain a powder form preparation for pernasal administration having the following formula.

| | |
|---|---|
| Erythropoietin | 30% (w/w) |
| DEAE-dextran | 30% (w/w) |
| HPMC | 10% (w/w) |
| Sucrose | 15% (w/w) |
| Buffer components | appropriate amounts |
| Total | 100% (w/w) |

### <EXAMPLE 23>

To a buffer solution containing erythropoietin, a buffer solution containing sucrose, chitosan and hydroxypropylmethyl cellulose (HPMC) was added. The resultant mixture was spray-dried to thereby obtain a powder form preparation for pernasal administration having the following formula.

| | |
|---|---|
| Erythropoietin | 30% (w/w) |
| Chitosan | 30% (w/w) |
| HPMC | 10% (w/w) |
| Sucrose | 15% (w/w) |
| Buffer components | appropriate amounts |
| Total | 100% (w/w) |

### <COMPARATIVE EXAMPLE 7>

Erythropoietin was dissolved in a buffer solution to prepare a liquid preparation for subcutaneous administration having the following concentration.

| | |
|---|---|
| Erythropoietin | 250 µg/ml |

### <EXPERIMENTAL EXAMPLE 9>

The preparations from Examples 20-23 and Comparative Example 6 were administered to male beagles through the nose; and the preparation from Comparative Example 7 was administered to male beagles subcutaneously. For the pernasal administration group, individual preparations were packed in gelatin capsules such that each capsule would give the dog 120 µg of erythropoietin per kg of body weight. The gelatin capsule was mounted in Publizer™ (Ishikawa Seisakusho) having a silicone tube about 5.0 cm in length bonded to its tip. Then, the silicone tube portion was inserted into the nasal cavity of each dog through one of the nostrils, followed by pressing the rubber ball portion of the Publizer™ to administer the preparation. In the subcutaneous administration group, the preparation from Comparative Example 7 was administered subcutaneously to the back of each dog such that 5 µg of erythropoietin was administered per kg of body weight. After the administration, blood samples were taken from the forearm vein at regular intervals. Blood erythropoietin levels were determined by ELISA. Table 9 shows the value of area under the blood erythropoietin level vs time curve (AUC) for each preparation. As a result, it was found that Eudragit E100 remarkably promotes the pernasal absorption of erythropoietin. Further, its absorption promoting effect was superior to the effect of poly-L-arginine, DEAE-dextran and chitosan. The bioavailability of the erythropoietin in the preparation of Example 20 was 15% relative as for the subcutaneous administration of erythropoietin.

**Table 9.**

| Preparation administered | Example 20 | Comparative Example 6 | Example 21 |
|---|---|---|---|
| AUC 0 → 11 hr (U · hr · ml⁻¹) | 29.1 | 1.5 | 19.1 |

| Preparation administered | Example 22 | Example 23 | Comparative Example 7 |
|---|---|---|---|
| AUC 0 → 11 hr (U · hr · ml⁻¹) | 6.6 | 20.2 | 7.9 |

| | | | |
|---|---|---|---|
| (1 µg of erythropoietin is equivalent to 130 U.) | | | |

### <EXAMPLE 24>

To a buffer solution containing growth hormone, a buffer solution containing D-mannitol and aminoalkylmethacrylate copolymer E (Eudragit E100) was added. The resultant mixture was spray-dried to thereby obtain a powder form preparation for pernasal administration having the following formula.

| | |
|---|---|
| Growth hormone | 10.0% (w/w) |
| Eudragit E100 | 7.5% (w/w) |
| D-mannitol | 75.2% (w/w) |
| Buffer components | appropriate amounts |
| Total | 100% (w/w) |

### <COMPARATIVE EXAMPLE 8>

To a buffer solution containing growth hormone, a buffer solution containing D-mannitol was added. The resultant mixture was spray-dried to thereby obtain a powder form preparation for pernasal administration having the following formula.

| | |
|---|---|
| Growth hormone | 10.0% (w/w) |
| D-mannitol | 82.7% (w/w) |
| Buffer components | appropriate amounts |
| Total | 100% (w/w) |

### <EXPERIMENTAL EXAMPLE 10>

Male beagles were used in this experiment. The preparations from Example 24 and Comparative Example 8 were separately packed in gelatin capsules such that each capsule would give the dog 50 µg of growth hormone per kg of body weight. The gelatin capsule was mounted in Publizer™ (Ishikawa Seisakusho) having a silicone tube about 5.0 cm in length bonded to its tip. Then, the silicone tube portion was inserted into the nasal cavity of each dog through one of the nostrils, followed by pressing the rubber ball portion of the Publizer™ to administer the preparation. After the administration, blood samples were taken from the forearm vein at regular intervals. Blood growth hormone levels were determined by ELISA. Table 10 shows the values of area under the blood growth hormone level vs time curve (AUC) for the preparations tested. As a result, it was found that Eudragit E100 remarkably promotes the pernasal absorption of growth hormone. The absorption ratio for the case where Eudragit E100 was added was 10 times higher than the ratio for the absence of Eudragit E100.

**Table 10.**

| Preparation administered | Example 24 | Comparative Ex. 8 |
|---|---|---|
| AUC 0 → 1 hr (ng · hr · ml⁻¹) | 13.0 | 1.3 |

### <EXAMPLE 25>

To a buffer solution containing influenza A antigen, a buffer solution containing D-mannitol and aminoalkylmethacrylate copolymer E (Eudragit E100) was added. The resultant mixture was spray-dried to thereby obtain a powder form preparation for pernasal administration having the formula below. It should be noted here that the percent by weight of influenza A antigen mentioned below is a value including the buffer components contained in the relevant reagent.

| | |
|---|---|
| Influenza A antigen | 4.0% (w/w) |
| Eudragit E100 | 7.5% (w/w) |
| D-mannitol | 81.2% (w/w) |
| Buffer components | appropriate amounts |
| Total | 100% (w/w) |

### <COMPARATIVE EXAMPLE 9>

To a buffer solution containing influenza A antigen, a buffer solution containing D-mannitol was added. The resultant mixture was spray-dried to thereby obtain a powder form preparation for pernasal administration having the formula below. It should be noted here that the percent by weight of influenza A antigen mentioned below is a value including the buffer components contained in the relevant reagent.

| | |
|---|---|
| Influenza A antigen | 4.0% (w/w) |
| D-mannitol | 88.7% (w/w) |
| Buffer components | appropriate amounts |
| Total | 100% (w/w) |

### <EXAMPLE 26>

To a buffer solution containing influenza A antigen, a buffer solution containing D-mannitol and poly-L-arginine was added. The resultant mixture was spray-dried to thereby obtain a powder form preparation for pernasal administration having the formula below. It should be noted here that the percent by weight of influenza A antigen mentioned below is a value including the buffer components contained in the relevant reagent.

| | |
|---|---|
| Influenza A antigen | 4.0% (w/w) |
| Poly-L-arginine | 7.5% (w/w) |
| D-mannitol | 81.2% (w/w) |
| Buffer components | appropriate amounts |
| Total | 100% (w/w) |

### <EXAMPLE 27>

To a buffer solution containing influenza A antigen, a buffer solution containing D-mannitol and diethylaminoethyl (DEAE)-dextran was added. The resultant mixture was spray-dried to thereby obtain a powder form preparation for pernasal administration having the formula below. It should be noted here that the percent by weight of influenza A antigen mentioned below is a value including the buffer components contained in the relevant reagent.

| | |
|---|---|
| Influenza A antigen | 4.0% (w/w) |
| DEAE-dextran | 7.5% (w/w) |
| D-mannitol | 81.2% (w/w) |
| Buffer components | appropriate amounts |
| Total | 100% (w/w) |

### <EXAMPLE 28>

To a buffer solution containing influenza A antigen, a buffer solution containing D-mannitol and chitosan was added. The resultant mixture was spray-dried to thereby obtain a powder form preparation for pernasal administration having the formula below. It should be noted here that the percent by weight of influenza A antigen mentioned below is a value including the buffer components contained in the relevant reagent.

| | |
|---|---|
| Influenza A antigen | 4.0% (w/w) |
| Chitosan | 7.5% (w/w) |
| D-mannitol | 81.2% (w/w) |
| Buffer components | appropriate amounts |
| Total | 100% (w/w) |

### <EXPERIMENTAL EXAMPLE 11>

### <<Day 1 of the Experiment (1st Administration)>>

Blood samples were taken from the forearm vein of beagles to be used in the experiment. The preparations from Examples 25-28 and Comparative Example 9 were packed separately in gelatin capsules such that 24 µl of influenza A antigen would be administered per one capsule. The gelatin capsule was mounted in Publizer™ (Ishikawa Seisakusho) having a silicone tube about 5.0 cm in length bonded to its tip. Then, the silicone tube portion was inserted into the nasal cavity of each dog through one of the nostrils, followed by pressing the rubber ball portion of the Publizer™ to administer the preparation.

### <<Day 15 of the Experiment (2nd Administration)>>

The preparations from Examples 25-28 and Comparative Example 9 were administered to the beagles through the nose. The grouping of the dogs, the dose and the method of administration were the same as for day 1 of the experiment.

### <<Day 29 of the Experiment>>

Blood samples were taken from the forearm vein of the beagles administered with influenza A antigen.

### <<Determination of the Amounts of Antibodies>>

The amounts of anti-influenza A antibodies in the sera collected on days 1 and 29 were determined by ELISA. The amounts of antibodies of the two subclasses, IgG1 and IgG2, were determined. Changes in the amounts of anti-influenza A antibodies based on their amounts on day 1 were compared. The amounts of anti-influenza A antibodies were compared as difference in absorbance between wells immobilizing the relevant antigen and wells not immobilizing the antigen. Tables 11 and 12 show the ratios of those individuals on day 29 in which anti-influenza A antibodies were induced (number of dogs: 4 in each group). As a result, it was found that both anti-influenza A-IgG1 and anti-influenza A-IgG2 are most frequently induced in the Eudragit E100-added group. From the above, it became evident that Eudragit E100 is useful as an adjuvant for pernasal vaccines and that the effect thereof is superior to the effect of the other polycations, i.e. poly-L-arginine, DEAE-dextran and chitosan.

| Table 11. Anti-Influenza A - IgG1 Antibody Induction Ratio | | | |
|---|---|---|---|
| Preparation administered | Example 25 | Comparative Example 9 | Example 26 |
| Day 29 (after two sensitizations) | 50% | 0% | 25% |

**Preparation**

| administered | Example 27 | Example 28 |
|---|---|---|
| Day 29 (after two sensitizations) | 0% | 50% |

| Table 12. Anti-Influenza A-IqG2 Antibody Induction Ratio | | | |
|---|---|---|---|
| Preparation administered | Example 25 | Comparative Example 9 | Example 26 |
| Day 29 (after two sensitizations) | 100% | 25% | 100% |

| Preparation administered | Example 27 | Example 28 | |
|---|---|---|---|
| Day 29 (after two sensitizations) | 25% | 25% | |

### Industrial Applicability

By adding a cationic polymer (in particular, a copolymer of aminoalkylmethacrylate or polyvinyl acetal diethylaminoacetate) or a cationic polymer plus a viscous polymer to a medicine of high molecular weight for producing a preparation in powder form, it is possible to achieve effective absorption of the medicine of high molecular weight through mucosa.

All of the publications, patents and patent applications cited in the present specification are incorporated herein by reference in their entirety.

## Claims

1. A preparation in powder form for administration through mucosa, comprising a medicine of high molecular weight and a cationic polymer.

2. The preparation of claim 1, further comprising a viscous polymer.

3. The preparation of claim 1, wherein the cationic polymer is a copolymer of aminoalkylmethacrylate.

4. The preparation of claim 1, wherein the cationic polymer is polyvinyl acetal diethylaminoacetate.

5. The preparation of claim 1, wherein the cationic polymer is poly-L-arginine.

6. The preparation of claim 2, wherein the viscous polymer is hydroxypropylmethyl cellulose.

7. The preparation of claim 1, wherein the medicine of high molecular weight is selected from the group consisting of bioactive peptides and proteins, antibodies, vaccines, and antigens.

8. The preparation of claim 7, wherein the bioactive peptide is a granulocyte colony-stimulating factor.

9. The preparation of any one of claims 1-8, which is a preparation for pernasal administration.

10. A pharmaceutical composition in powder form, comprising a medicine of high molecular weight and a cationic polymer.

11. The pharmaceutical composition of claim 10, wherein the medicine of high molecular weight is selected from the group consisting of bioactive peptides and proteins, antibodies, vaccines, and antigens.

12. The pharmaceutical composition of claim 11, wherein the medicine of high molecular weight is selected from the group consisting of insulin, erythropoietin, growth hormone, and influenza antigens.
